(19) Europäisches Patentamt / European Patent Office / Office européen des brevets

(11) **EP 4 309 639 A2**

(12) **EUROPEAN PATENT APPLICATION**

(43) Date of publication:
**24.01.2024 Bulletin 2024/04**

(21) Application number: **23214685.2**

(22) Date of filing: **14.06.2012**

(51) International Patent Classification (IPC):
**A61H 39/08** $^{(2006.01)}$

(52) Cooperative Patent Classification (CPC):
**A61B 18/1477; A61H 39/002; A61H 39/086;**
**A61N 1/06; A61N 1/328;** A61B 2018/00452;
A61B 2018/0047; A61B 2018/00589;
A61B 2018/00726; A61B 2018/00732;
A61B 2018/00767; A61B 2018/126;
A61B 2018/143; A61B 2018/1475

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**

(30) Priority: **14.06.2011 KR 20110057691**
**14.06.2011 KR 20110057692**

(62) Document number(s) of the earlier application(s) in accordance with Art. 76 EPC:
**12800448.8 / 2 722 072**

(71) Applicant: **ViOL Co., Ltd.**
**Seongnam-si, Gyeonggi-do, 13510 (KR)**

(72) Inventors:
• **NA, Jong Ju**
  **138-836 Seoul (KR)**
• **GWAK, Jeong Gu**
  **138-836 Seoul (KR)**

(74) Representative: **Meissner Bolte Partnerschaft mbB**
**Widenmayerstrasse 47**
**80538 München (DE)**

Remarks:
This application was filed on 06-12-2023 as a divisional application to the application mentioned under INID code 62.

(54) **APPARATUS AND METHOD FOR IMPROVING SKIN USING A RA-EFFECT OR RA PLUS-EFFECT**

(57) The present invention relates to a skin treatment apparatus comprising:
- a plurality of bipolar needles (300) with sharp ends (301);
- a fixture (100) into which the needles (300) are embedded;
- a drive module for transferring power to the fixture (300) directly or indirectly for the needles (300) to be inserted into a skin; and
- an electric energy transmission module electrically coupled to the needles (300),
wherein the electric energy transmission module is configured to provide the plurality of needles (300) with an electricity which is an alternating current having a high frequency.

[Figure 5]

**Description**

**Technical Field**

The present invention is for skin treatment using electricity energy, especially with the primary purpose for skin treatment for beauty, and in some cases, can be used as a direct instrument or supplement for skin cosmetic surgery.
Specifically, The present invention is to give treatment for firm skin by the method and device using high frequency, which minimizes skin aging by directly stimulating dermis between epidermis and subcutaneous layer and guarantees skin improvement.

**Background Art**

Generally, skin is the largest protection organ that covers the whole body and maintains homeostasis on surface of the body with three layers consisting of epidermis, dermis, and subcutaneous layer.
Dermis is the layer beneath epidermis, directly connected to stratum basal, and mostly made up of dermis tissue, protein, glucide, glycosaminoglycans, mineral, inorganic salt, etc. in gelatinous form, and dermis consists of capillary that provides nutrition to epidermis and circulates blood, papillary layer where lymphatic duct that carries lymph is located, and reticular layer which is made up of collagenous fiber related to skin wrinkle, collagen, elastic fiber that gives elasticity to skin, elastin, and the substrate(water storage). Subcutaneous layer lies below dermis, contains plenty of fat, and attaches the skin to underlying muscle and bone at the bottom of the skin.
One of the most popular treatments to prevent skin aging is massaging with nutritious substance. However, although this type of skin treatment of massaging provides nutrition to epidermis and make the skin glow, it does not last for long and rarely show the improvement in a short period of time.
So a new way was presented by using a syringe to inject serum into the skin. However, this way costs much for treatment and causes side effects depending on unique constitution of each individual, resulting in adverse effect on skin.
After that, the new method developed that delivers high frequency indirectly from epidermis to dermis. However, for it is transmitted to dermis through epidermis, it is not very effective even though it uses high voltage. Also, another problem was that patient needed to receive continuous treatment to obtain effective result and it was costly.
Later, revitalizing regeneration of collagen by wounding dermis was devised as skin improvement method. And inserting a needle (The present invention refers it as a pin, which is the same thing in principle) into dermis to give electric stimulation was invented as one of those methods.
Taking a detailed look at this, dermis is composed of protein called collagen. Collagen is triple helical structure including fibroblast and polypeptide and when heat is applied to collagen tissue, physical change of protein matrix part occurs at its shrinkage temperature. Soft tissue remodeling, which is one of these changes, is a phenomenon happening at cellular and molecular level, and the triple helical binding is disassembled by partial denaturalization of collagen or shrinkage caused by artificial heat resulting in destruction of molecular bindings of matrix. When cell shrinkage occurs, collagen lies on lower part as the static supporting matrix within tightened soft tissue. The initial deposition and subsequent remodeling of scar matrix offers a way to modify the shape and consistency of soft tissue for beauty purpose. Soft tissue remodeling is immediate process compared to the shrinkage appearing as a result of movement of fibroblast cell or natural healing. Ultimately, the treatment with cosmetic purpose such as skin improvement or wrinkle improvement can be considered to begin by applying various energy sources to selected area and solidify the tissue.
When energy is applied to dermis, the form of effects on dermis differs depending on the amount of applied energy, and it is generally divided into three phenomena: carbonization, vaporization, and coagulation. Na-Effect of this device improves skin condition by forming coagulation condition in principle, and it can also be expressed with such phrases as "to burn", " to apply heat", "to raise temperature", even though none of these may be exact and typical description. However, for The present invention, "to raise temperature" would be the most suitable description conveying The present invention's feature by applying energy to achieve coagulation so "to raise temperature" could be used to express a way to achieve coagulation.
The term "to raise temperature" can be perceived as applying energy, but the meaning of "to raise temperature" written on the claims for The present invention was used as "to apply energy to achieve coagulation"
One of the existing technologies related to this is the patent 2000-0058346. This device was invented to restore the wrinkle back to flat skin, and is designed to verify wrinkle's thickness, length, shape, and etc., set pulse, time, and watt of elevation current, which magnifies the cell tissue on control device box wrinkle treatment device, and determine the needle length according to the length and depth of wrinkle so the patient can remove the wrinkle after installing needle on the hand piece.
The patented formula had one needle which was not able to show the principle enough, and especially it was at very dangerous level to apply it to human body. Later, this becomes mother formula for various patents, and one of those is the patent 10-0856736 by the inventor of The present invention. Especially, The present invention became a milestone for increasing the effectiveness of skin treatment for dozens of needles penetrate skin simultaneously.

Another similar technology invented recently is the domestic patent 10-2009-23494. If you look at this more closely, it is about using high frequency to give treatment for elastic skin. In more details, it revitalizes cell tissues to maintain elastic skin to minimize skin aging by directly delivering high frequency into dermis between epidermis and subcutaneous layer. This skin treatment device has a pair of electrodes inserted in the main body case to contact the above electrode that the connection part where the current runs is inserted and fixed. It uses high frequency which is composed by a number of high frequency generation terminals generating high frequency currents when the live high frequency inserted and fixed in the lower part of above connection part is inserted in reticular layer which composes dermis of skin.

However, although this skin treatment method was effective in that it could see result within a short period of time for it inserts energy directly to dermis compared to the previous massage, laser method, or using high frequency that does not penetrate skin with needle, there were many side effects as well.

The biggest side effect was it burns not only dermis and but also epidermis between needles that epidermis is damaged and skin was burned permanently quite often.

In case of searing dermis properly, coagulation boosts generation of collagen. However, if epidermis is burned by the treatment above, the area becomes scar and the treatment for skin improvement may tend to bring more damage.

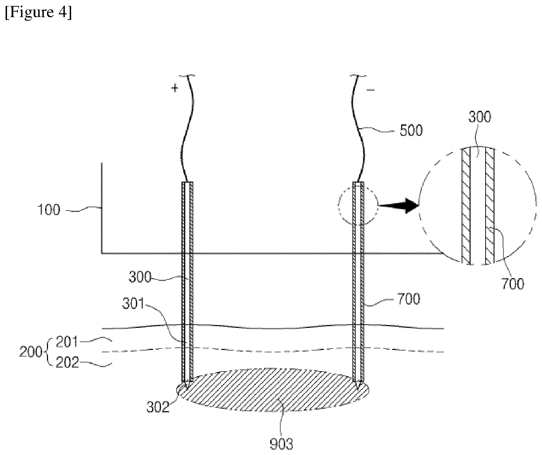

This phenomenon that burns both dermis and epidermis between needles was very natural in the aspect of electric engineering. People thought electric energy flows between the needles that act as electrodes and the skin has much lower resistance than air, yet it does have consistent resistance which generates heat. And it was indeed happening. (See figure 4)

Another problem of treatment above is that fine control was very difficult. Because as stated above, in case they are heated, the forms are quite drastic. Therefore, it was considerably difficult to treat in several times according to the skin tissue status. In terms of being a medical device, it brings a serious problem restricting safety.

So since then, most of technology was developed to have insulated coating on the needle except the part penetrating dermis. However, this caused many problems again.

First, the needle becomes thicker when coating and it caused some problems. The major problems included leaving a big scar, more pain when inserting the needle onto skin, and possibility of side effect if the patient is allergic to insulating coating material.

Second problem was there was always high possibility that coating would come off. This was happening without a doubt considering the device was continuously penetrating skin. In case that coating comes off, it could cause bigger side effects than being careful without coating from the beginning.

Third problem was that the device was still quite extreme burning the skin between uncoated needles. It was hard to do fine control and had possibility to burn the skin

Another example of previously existing technology is device using monopole. Monopole is a form of treatment that attaches an electrode on the target area and the other on any random area. However to see the result with this method, there was a serious flaw that it had to flow high voltage electricity to generate heat. The usage was limited due to not only the danger of burn, but also the potential risk for internal organs by authorized current that can even lead someone to death.

This device was invented during the process of researching and trying to solve these problems.

**Disclosure**

**Technical Problem**

The technical problem that this device is trying to resolve are the problems of previous technology as mentioned above.

**Technical Solution**

The present invention was invented by adopting compositions that could never be imagined for previously existing technology and creating the revolutionary result through innovation. This new result of The present invention was named "Na-Effect". In addition to Na-Effect, the inventor continued to research and gained another result, which are the examples shown in drawing 8 and drawing 10. These effects are named Na-Plus effect on The present invention.

In principle, Na-Effect and Na-Plus effect use not insulated coating needles only in any cases; it is recommended that the tip of the needle is not coated. The Na-Effect and Na-Plus effect contributes significantly to offer various treatment methods to skin care device according to its distinctive applications.

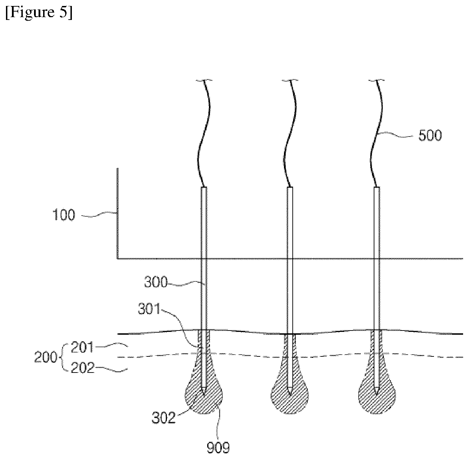

Heated areas of Na-Effect are different from those of existing bipolar electrodes employing system. The temperature is raised and the heat is concentrated at the area in dermis for coagulations. The heat minimally affects epidermis area. Also, the heated area around the end of tip of needles is shaped like an oval or tear shaped as shown in FIG. 5.

Also, Na-Effect is different from the existing technology that the heat is concentrated between needles. In Na-Effect, the heated area is around each needle. In addition, Na effect is invented based on countless trials and the results definitely improve previously existing problems.

First of all, Na-Effect is the method that burns the area around a tip of each electrode separately rather than burning the space between needles. Therefore this is a revolutionary method that can adjust different factors of electricity or space and have some fine control for treatment. In other words, the technique has significantly improved compared to the existing techniques that a doctor can adjust the number of treatment as needed for different area by the strength.

In addition, since Na-Effect can affect only dermis without burning epidermis, it has a huge difference from previously existing technology that had coating on the needle, and completely solve the problem of insulation coating. Therefore, treatment using The present invention by Na-Effect, in principle, can be done without coating the needle. Sometimes, even when treating patient under somewhat severe condition using application of Na-Effect, only tip part of the needle needs to be coated, and normally there is no need to do so.

The required composition for Na-Effect and Na-Plus effect is bipolar, AC and high frequency. The space between electrodes is important for Na-Effect. Because the space between electrodes is too tight, the tip of the each needle where it is heated may stick to each other preventing Na-Effect. However, Na-Plus effect is not limited by space since it can work even with narrow space.

Another composition of Na-Effect and Na-Plus effect is that needle should not be coated. This is different from previously existing technology trend. This doesn't mean the needle cannot be coated but what was necessary for previously existing technology became unnecessary. This shows the significant difference from previously existing technology that coating prevents the product from functioning properly.

Another composition related to Na-Effect and Na-Plus effect is that number of needle ought to form a net when link the tip of needles. Theoretically, it works if the number of needles exceeds four. However, it is desirable to have at least four needles on each side assuming it is a quadrangle.

Another composition of Na-Effect and Na-Plus effect is to authorize current on needles yet it is recommended that polarity of neighboring needles must be opposite as (+) and (-).

Advantageous Effects

The greatest effect of The present invention is that this can solve the critical problems of previous technology.

First, the biggest side effect of previously existing technology that burns both dermis and epidermis between needles and that burns skin too much causing permanent damages can be solved when using any device applying Na-Effect or Na-Plus effect

Na-Effect is totally different from the form of previously existing bipolar electrode that burned skin, and heat is applied only at the area near dermis centered on the tip of the needle in form of light bulb, as shown in drawing 6. Therefore, it is a revolutionary method with significant improvement compared to the techniques in the past that can adjust different factors of electricity or space and have some fine control for treatment, or just keep the strength weak so a doctor can adjust the number of treatment as needed for different area by the strength. This is a revolutionary change without a doubt in medical aspect. This is the same for Na-Plus Effect.

Also, previously existing technology had a tendency that the burned epidermis area becomes scar right away and an action for skin improvement causes greater skin damage. As shown in the drawing, another major effect is that epidermis burn rarely happens.

So, except the tip of the needles where it reaches to dermis, most of technology insulated the needle and give the treatment since then. However, The present invention had disadvantage of not being able to utilize Na-Effect or Na-Plus Effect if it is coated, which solved the problem of coating that thicker needle leaves bigger scar and greater pain and it may cause worse side effect when the coating comes off.

**Description of Drawings**

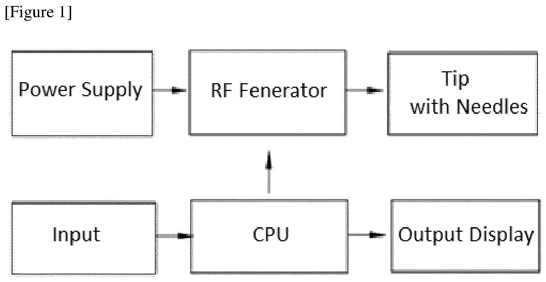

Figure 1 is a general structural map for application of this device

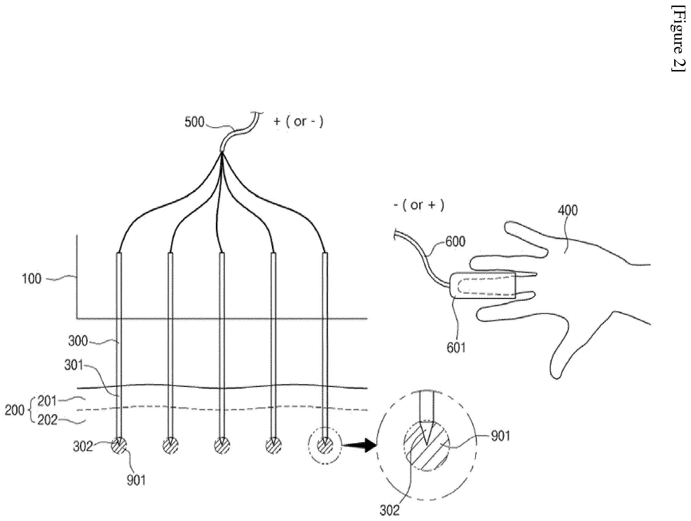

Figure 2 is a demonstration of treatment result in case of using monopole for treatment

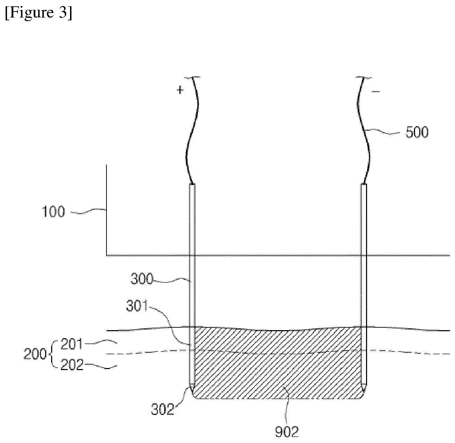

Figure 3 is the treatment result indication when using the bipolar electrode without insulation coating on existing needle.
Figure 4 is the treatment result indication when using the bipolar electrode with insulation coating on existing needle except the tip of the needle.

Figure 5 is the treatment result indication when using the needle without coating for Na-Effect on skin insertion part

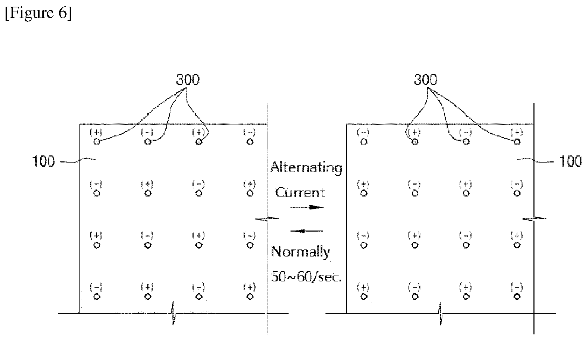

Figure 6 is an application example for change of electrodes in current realizing Na-Effect

Figure 7 is the treatment result indication as a practical application realizing Na-Plus effect when giving treatment

Figure 8 is another treatment result indication as a practical application realizing Na-Plus effect when giving treatment

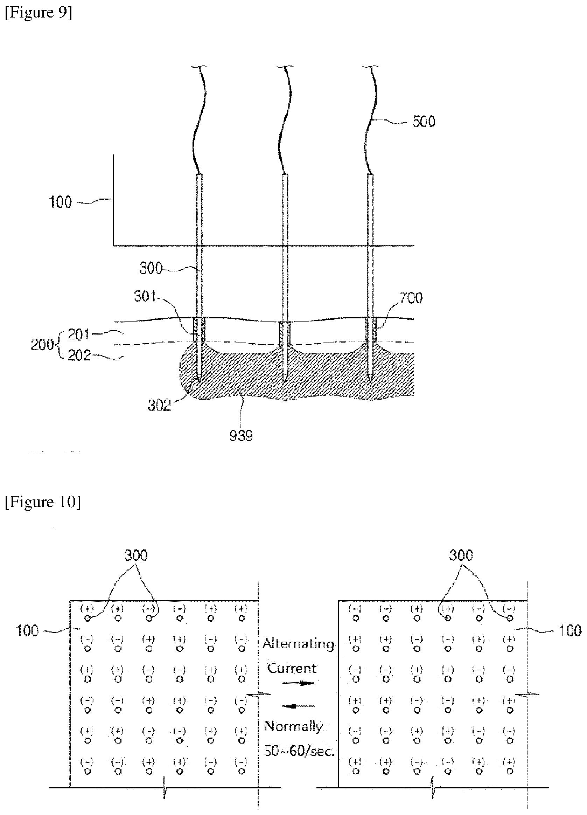

Figure 9 is another treatment result indication as a practical application realizing Na-Plus effect when giving treatment

Figure 10 is another application example for change of electrodes in current realizing Na-Effect

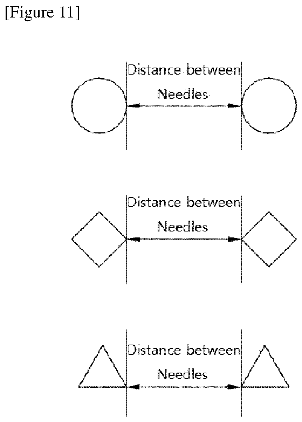

Figure 11 is a picture of the space between a needle and another one.

<Explanation of symbols referring to major sections of the Figures>

100: needle fixing part 200: skin

201: epidermis 202: dermis

300: pin/needle/electrode 301: pin point penetrating skin

302: pin point penetrating into dermis

400: patient's hand receiving treatment using monopolar electrode

500, 600: electricity access line

700: insulation coating section

901: the picture of seared area when using monopole electrode

902: the picture of burning area when using an uncoated bipolar electrode on existing needle

903: the picture of burning area when using a coated bipolar electrode except on the tip of the existing needle

909: the picture of the uncoated needle used on penetration spot and the picture of treated skin after Na-Effect or Na-Plus effect takes effect

**Mode for Invention**

The present invention went through various experiments based on what was mentioned above, and the following is the result in details based mainly on the composition.

The present invention pursues two effect, Na-Effect and Na-Plus effect. Fundamentally, having their foundation on Na-Effect, these two effects are related to each other, and the difference between two are that in a range where Na-effect takes place by voltage, current, authorization time, needle thickness, resistance, current frequency, high frequency, conductivity, needle depth penetrating skin, etc., Na-Plus effect tends to appear as it becomes more severe. Therefore, technological compositions representing both effects are quite similar.

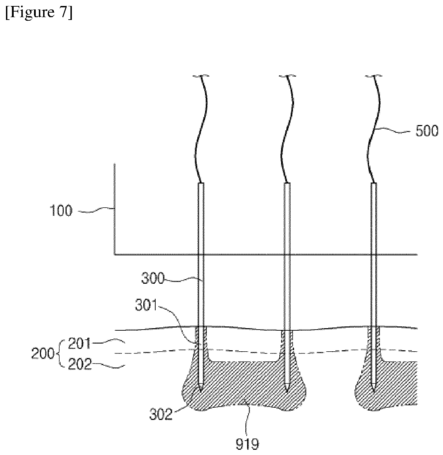

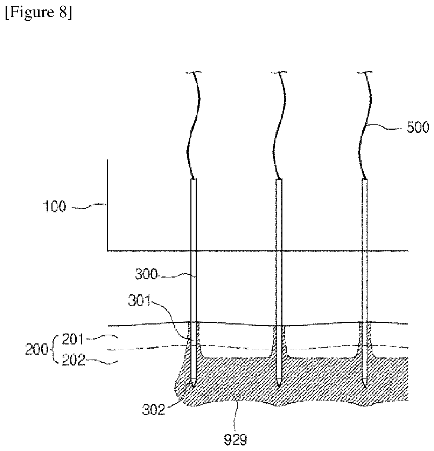

The required composition for Na-Effect and Na-Plus effect is bipolarity, AC and high frequency. The distance between electrodes is important for Na-Effect and Na-Plus effect. For Na-Effect, it is not desired to have needles in proximity to each other because if the distance between electrodes is too close, the heated area of each needle will be connected. However, it does not affect the Na-Plus effect. Na-Plus Effect happens when the heated areas of Na-Effect in dermis expand and are connected in dermis, not in epidermis as shown in Fig. 7 and 8.

Alternating current (AC) is the current that changes magnitude and direction periodically and usually abbreviated as AC (alternating current). Most typical type is sine wave and it can change to triangular or square wave. It has different characteristics from direct current with one direction. In general, AC is used with uniform frequency in different countries at 50Hz or 60Hz

AC is the most critical factor for generation Na-Effect and Na-Plus effect. When the frequency is great, unless it is especially high (hundreds Hz), it is considered it will not be problematic. Even when the frequency is pretty high, narrowing the needle space can cancel the high frequency. However, low frequency will cause problem to operate Na-Effect. Also with this case, if above 20Hz, Na-Effect can be realized by controlling other factors. We will discuss control of other factors later again.

Also inspected and experimented from various angle, Na-Effect and Na-Plus effect are not realized if it's not high frequency. For example, Na-Effect and Na-Plus effect was not generated with low frequency, ultrasonic waves, medium frequency, ion, etc.

Especially, within the range of high frequency above 0.5 (MHz), Na-Effect and Na-Plus effect was displayed. And the optimal range is being used currently, which was determined to be around 2 MHz through experiments. In other words, the desired range is 0.5 - 10 MHz, more desired range is 1 - 4 MHz, and the optimal range is 1.5 - 2.5 MHz. However, depending on a given condition, it is expected to vary although it does not get out of the average range given above.

To explain this again in depth, if you do perform electricity conductivity experiment for dry skin and wet skin with 2 MHz and 1 MHz, dry skin has 0.037102 at 2 MHz and 0.013237 at 1 MHz, wet skin has 0.26649 at 2MHz and 0.2214 at 1 MHz. Consequently, flow of RFenergy is smoother at 2 MHz, and the same energy is applied on dry skin, the probability for spark at 1MHz can be varied by other variables, but when comparing arithmetical electricity conductivity, it is three times higher.

To describe this theoretically in detail, high frequency creates energy field to relatively closer area when the frequency is higher with the same current, and when the frequency is low, it creates broader energy field from the electrode.

For example, if below 0.5MHz, even though the electrodes are far away from each other, energy area is too broad that it may burn even epidermis causing scar. Especially, skin treatment on small area such as eyelid can affect eye. Meanwhile, for over 10MHz, energy area is too narrow so treatment time increases and it is also difficult to create energy area for best treatment.

Next, Na-Effect and Na-Plus effect is deeply related to the space between needles. Especially the space of needle is related to all the other compositions as well. For example, when high frequency increases, needle space decreases, and when high frequency decreases, space increases according to the experiment. In case of 2 MHz, the optimal needle space is proved to be around 2mm through experiments. We will discuss this in detail later

Next important composition that affects Na-Effect and Na-Plus effect is whether the needle is insulated and coated or not. In principle, needles don't need to be insulated since epidermis barely gets burned where Na-Effect and Na-Plus effect work, and only around the tip of the needle that penetrates into dermis as shown drawing 6,8,9,10 is seared like the shape of light bulb, or similar shape. However, it cannot be said the coating itself obstruct Na-effect and Na-plus effect unless it is for the purpose of electricity insulation. For example, there were cases that metal coating or coating by heat to increase the degree of strength of the needle didn't affect Na-Effect and Na-Plus effect.

In other words, to utilize Na-Effect and Na-Plus effect to their maximum, it is desirable not to insulate, and even if you do, it is better not to insulate dermis section at least. However, it doesn't mean that Na-Effect and Na-Plus effect does not take place when the needle is insulated. For example, when the needle is insulated except on its tip, even if it may have similar form of burning area, but it is completely different result from significantly different process.

Another composition closely related to Na-Effect and Na-Plus effect is the arrangement of needle, the arrangement of electrodes. In principle, Na-Effect and Na-Plus effect authorizes current on needle, but neighboring needles should be arranged that they will have opposite polarity as drawing 7. This arrangement is made up of squares, and on each corner needle is located, with opposite polarity for the closest needle. Of course, since AC is used, each needle changes its polarity 50-60 times a second in case AC electricity. Even with that, it is possible to arrange the neighboring needles that authorizes currents every moment to have opposite polarity as drawing shown in 7. This is the principle of The present invention that each electrode has opposite polarity with its closest neighboring needles crossing (+) and (-) in each direction.

In general, it was verified that the square is desired, yet it can be any shape of tilted rhombus, rectangle, or quadrangle with different side lengths, etc. as long as the neighboring electrodes are arranged differently to cause Na-effect or Na-plus effect.

Also, in some cases, non-conductive needle can be laid out at places, or remove needles on purpose. However, the principle of arranging polarity is the standard to have opposite polarity with the closest neighboring needles crossing (+), (-) in opposite direction, which certainly contributes to Na-Effect or Na-Plus Effect and select the composition. The meaning of "in principle" in The present invention regarding the arrangement of polarity is used to include the occasions mentioned above.

Another example of arrangement allowing Na-Effect and Na-Plus effect found is drawing 11. Assuming a needle is A as shown in drawing 11, if one of the directions with closest needle has the same polarity as A, Na-Effect or Na-Plus effect could be realized.

Also, it is unavoidable to combining different shapes when designing a product. For this device, it is possible to have various setting including most scenarios that made the polarity of neighboring electrode differ from each other to obtain Na-Effect or Na-Plus effect.

Minimum number of needle for this is 4, but Na-Effect or Na-Plus effect can be partly operated as at least more than nine parts include the identical arrangement.

The major part refers to occasions as arranging electrodes of certain area as above to utilize Na-effect or Na-Plus effect.

Another important composition is voltage and it is not only a critical consideration to determine needle space, but also directly related to safety of the device. Therefore, normally it is measured based on voltage authorized on skin.

Another important composition is voltage and it is not only a critical consideration to determine needle space, but also directly related to safety of the device. Therefore, normally it is recommended to measure the authorized voltage focusing on voltage authorized on skin.

Therefore, the meaning of "the authorized voltage on skin" and the observation of the possibility of measurement are as following:

The actual voltage authorized for skin is the voltage applied between the surface of a needle and contacting are of skin. Therefore, different from the authorized voltage on the device, it can vary depending on the total amount of three resistance, device resistance, needle resistance, and skin resistance. Also, it can be the same or vary depending on the surface area of the needle. Because the skin resistance can be different for certain area of needle surface, so even applying the same voltage would result in different voltage on different area of skin.

However, even though there is difference in resistance within skin, it's not much differences, The present invention measured and stated the voltage on the surface of needle with the needle penetrating the skin.

This is directly associated composition with authorized amount of energy and must not go over 100V. Recommended voltage is 10 - 60V, and optimal voltage is found to be 20 - 40V through experiments. Of course, Na-Effect or Na-Plus effect can be shown even when it goes over 100V. Therefore, under certain severe circumstance, it might be desirable to use 100V, but it is hard to set it to go beyond 100V because if voltage is set as 100V, Na-Effect or Na-Plus effect occurs but it may leave scar.

When giving treatment, it is possible to design for various voltages on skin according to the device voltage and circuit design. It is easy for the professional personnel to operate and is not a major feature of this device, so detailed explanation will not be given.

Another variable is current. However, this varies depending on voltage and resistance, so it is possible to compute the value if device resistance, needle resistance, and skin resistance value are set.

$$V = I * R$$

Resistance is divided into three resistance, device resistance, needle resistance, and skin resistance.

Another important composition is energy duration time. If you look at minimum duration, it takes 0.2 second for high frequency energy to reach stable area and if energy duration time is too short, the effect is barely measurable, you cannot shorten the time too much. It is indeed found that it should be at least 0.05 second based on the result of measured time. A major characteristic of The present invention includes comparably short energy duration time, however the result of measuring the longest time that shows effect is 0.8 seconds. Surely expanding the space between needles and minimize voltage would allow longer duration time. Nevertheless, under the optimal condition, the range would be 0.05-0.8 second.

However, desirable time lies between 0.1 - 0.4 seconds, and optimal time is measured as 0.1 - 0.2 seconds. It was much more difficult to find the time than expected, and required countless trials and errors with insight. Because only after Na-Effect was invented, it was possible to imagine having this short overload time using this high frequency and alternating currency with low voltage.

Indeed, you can verify that this device provide energy within much short period of time when you compare the electricity authorization of this device and the fact that device stimulating skin with electrode without penetrating skin with needle(previous Polaris device) provided energy for longer than 0.5 second at much higher voltage.

Next important composition is the length and thickness of the needle. The thickness of the needle affects the space between needles. In addition, it should have strength to endure the repeated process of penetrating it into skin and taking it out. Also, as the needle thickens, it may involve greater pain and bigger scar.

In consequences, the thickness of a needle must be optimized at a point where it will not bend when penetrating the skin yet minimize the pain and wound. Currently, thickness of a clinically used needle is either 0.25mm or 0.3mm and this size is considered moderate. Even if the needle thickness is out of this range mentioned above, it didn't affect much to show the Na-Effect or Na-Plus effect.

the length of a needle can be different based on the design of the device and is difficult to discuss uniformly. However, if focusing on the length inserting into skin, there is not a major problem to mention it based on the thickness of skin and treatment purpose.

Regarding this, Drawing 1 lists depth of skin layers and you can use the drawing to determine. In general, depth of epidermis is between 0.2mm and 1mm, and depth of dermis is between 1mm and 4mm. Therefore, the length to reach the top of dermis must be at least 0.2mm and the maximum length to reach the bottom of dermis is 5mm. And it can be said that desired depth for treatment is 1mm - 5mm considering safety. Therefore, total length of needle can vary based on the type of device, but the length of a needle being inserted onto skin is advised to be 1mm - 5mm.

Only thing is that, epidermis consists of stratum corneum, stratum granulosum, stratum spinosum, Stratum basale with different skin resistance level, and Na-Effect is visible with microscope, it is possible to give treatment solely for epidermis. At this time, the depth of treatment can be less than 1mm. Also, there is a case that the depth to reach subcutaneous layer is 6mm. Therefore the length of a needle can be designed diversified even for one skin layer within the range of 1mm - 6mm depending on penetration depths.

One of the most critical part to design a device to obtain Na-Effect and Na-Plus effect is the space between needles as mentioned above. There are many ways to measure the space, but here we will explain focusing on closest space between the surface of a needle and the closest needle as Drawing 12. Regarding The present invention, space between needles is defined as mentioned here.

We gained possible result up to 1.3 mm that allows Na-Effect and Na-Plus effect. Therefore, desirable space is observed to be around 1.3 - 3.0mm, although wider space is considered plausible if authorized current or resistance is decreased.

To explain this in detail again, there is a risk of scar or burn from unexpected excessive heat if the length is shorter than 1.3mm, or if the length is greater than 3.0mm, the time to build treatment area with same are for effective density increases, treatment time, increases and efficiency for treatment drops significantly, which also is a cause of financial burden to patients.

However, the space between needles can change depending on many variables. under the premise of gaining proper Na-Effect and Na-Plus effect, composition that affects the space between needles are as following:

First, if the energy increases or the needle becomes thicker, the space between the needle should be expanded. If installed separately, power is equivalent to voltage multiplied by current and time. Therefore the space between needles must be expanded if any one or two of voltage, current, overtime, or thickness of the needle increases.

However, even if any of resistance, AC frequency, high frequency, conductivity or the length of needle penetrating the skin increases, narrowing the space between needles according to the magnitude of effect may not affect Na-Effect and generating Na-Plus effect.

It can be expressed by the formula below.

$$\text{SPACE} = N \frac{(\text{Power } (=\text{Energy}))*(\text{Needle Thickness})*(\text{Conductivity})}{(\text{Resistance})*(\text{AC Frequency})*(\text{High Frequency})*(\text{Needle Depth Penetrating Skin})}$$

N: proportional factor
registance: device resistance, needle resistance, skin resistance(only shin resistance can be used when considering only inner skin condition)
J=W*t, so
power(=energy)=V*I*T, (W=V*I)
power(=energy)=I^2*R*T, (V=I*R)
SO,

$$\text{SPACE} = N \frac{V*I*t*(\text{Needle Thickness})*(\text{Conductivity})}{(\text{Resistance})*(\text{AC Frequency})*(\text{High Frequency})*(\text{Needle Depth Penetrating Skin})}$$

It can be expressed by the formula above also.

In the present invention, needle fixing part performs a function of preventing the scattering of the needle, nevertheless, and the needle can be substantially moved in the needle fixing part.

Aspects of the present invention include the following:

[Aspect 1]

Multiple needles with sharp ends;
A fixture that needles are embedded onto;
A drive system that drives movement directly or indirectly to the fixture for the above needles to be inserted into skin; and
An electric transmission that is connected to the above needles electrically is also included.
The applied electricity is alternating current, the form of signal is high frequency and the needles are bipolar, and this device aims to apply energy in a shape described in Na-effect to achieve coagulation on treated area of skin.

[Aspect 2]

Multiple needles with sharp ends;
A fixture that needles are embedded onto;
A drive system that drives movement directly or indirectly to the fixture for the above needles to be inserted into skin; and

An electric transmission that is connected to the above needles electrically is also included.

The applied electricity is alternating current and the needles are bipolar, the polarity of a minimum of 9 neighboring needles is different, alternating between (+) and (-), the form of signal is high frequency, and this device aims to apply energy in a shape described in Na-effect to achieve coagulation on treated area of skin.

[Aspect 3]

Multiple needles with sharp ends;

A fixture that needles are embedded onto;

A drive system that drives movement directly or indirectly to the fixture for the above needles to be inserted into skin; and

An electric transmission that is connected to the above needles electrically is also included.

The applied electricity is alternating current and the needles are bipolar, the closest distance between neighboring needles is between 1.3 - 3.0 mm, the form of signal is high frequency, and this device aims to apply energy in a shape described in Na-effect to achieve coagulation on treated area of skin.

[Aspect 4]

Multiple needles with sharp ends;

A fixture that needles are embedded onto;

A drive system that drives movement directly or indirectly to the fixture for the above needles to be inserted into skin; and

An electric transmission that is connected to the above needles electrically is also included.

The applied electricity is alternating current and the needles are bipolar, the closest distance between neighboring needles is between 1.3 - 3.0 mm, the polarity of a minimum of 9 neighboring needles is different, alternating between (+) and (-), the form of signal is high frequency, and this device aims to apply energy in a shape described in Na-effect to achieve coagulation on treated area of skin.

[Aspect 5]

Multiple needles with sharp ends;

A fixture that needles are embedded onto;

A drive system that drives movement directly or indirectly to the fixture for the above needles to be inserted into skin; and

An electric transmission that is connected to the above needles electrically is also included.

The applied electricity is alternating current, the frequency of alternating current is higher than 20Hz including 20Hz, the needles are bipolar, the closest distance between neighboring needles is between 1.3 - 3.0 mm, the needles in the dermis area are not insulated, the voltage applied to the needles is below 100V, including 100V, and the current flowing time through needles is between 0.05 to 0.8 seconds, and this device aims to apply energy in a shape described in Na-effect to achieve coagulation on treated area of skin.

[Aspect 6]

Multiple needles with sharp ends;

A fixture that needles are embedded onto;

A drive system that drives movement directly or indirectly to the fixture for the above needles to be inserted into skin; and

An electric transmission that is connected to the above needles electrically is also included.

The applied electricity is alternating current, and the needles are bipolar, the closest distance between neighboring needles is between 1.3 - 3.0 mm, the polarity of a minimum of 9 neighboring needles is different, alternating between (+) and (-), the form of signal is high frequency, and this device aims to apply energy in a shape described in Na-plus effect to achieve coagulation on treated area of skin.

[Aspect 7]

Multiple needles with sharp ends;

A fixture that needles are embedded onto;

A drive system that drives movement directly or indirectly to the fixture for the above needles to be inserted into skin; and

An electric transmission that is connected to the above needles electrically is also included.

The applied electricity is alternating current; the frequency of alternating current is higher than 20Hz including 20Hz. The needles are bipolar and the closest distance between neighboring needles is between 1.3 - 3.0 mm. The needles in the dermis area are not insulated, the applied volatge is below 100V, including 100V, the device has the current flowing time between 0.05 and 0.8 seconds, and this device aims to apply energy in a shape described in Na-plus effect to achieve coagulation on treated area of skin.

[Aspect 8]
Within aspects from 1 to 7, the device has the high frequency range of 0.5 - 10 MHz.

[Aspect 9]
Within aspects from 1 to 7, the device has the high frequency range of 1 - 4 MHz.

[Aspect 10]
Within aspects from 1 to 7, the device has the high frequency range of 1.5 - 2.5 MHz.

[Aspect 11]
Within aspects from 1 to 7, the device has needles with the maximum diameter of 0.25 mm or 0.3 mm. The penetration depth of needle into skin is 1 mm - 5 mm.

[Aspect 12]
Within aspects from 1 to 7, the device has needles with the maximum diameter of 0.25 mm or 0.3 mm. The penetration depth of needle into skin is 0.1 mm - 1 mm.

[Aspect 13]
Within aspects from 1 to 7, the device has the applied voltage of 10 - 60V.

[Aspect 14]
Within aspects from 1 to 7, the device has the applied voltage of 20 - 40V

[Aspect 15]
Within any aspects from 1 to 7, the device has the current flowing time between 0.1 and 0.4 seconds.

[Aspect 16]
Within any aspects from 1 to 7, the device has the current flowing time between 0.1 and 0.2 seconds.

**Claims**

1. A skin treatment apparatus comprising:

   - a plurality of bipolar needles (300) with sharp ends (301);
   - a fixture (100) into which the needles (300) are embedded;
   - a drive module for transferring power to the fixture (300) directly or indirectly for the needles (300) to be inserted into a skin; and
   - an electric energy transmission module electrically coupled to the needles (300),

   wherein the electric energy transmission module is configured to provide the plurality of needles (300) with an electricity which is an alternating current having a high frequency.

2. The skin treatment apparatus according to claim 1,
   wherein the apparatus is configured to achieve coagulation on a treated area of the skin according to Na-effect.

3. The skin treatment apparatus according to any one of the preceding claims, in particular to claim 2,
   wherein according to the Na-effect, an area around a tip (302) of each needle (300) is coagulated separately rather than a space between the needles (300).

4. The skin treatment apparatus according to any one of the preceding claims,

   wherein the apparatus is configured to achieve coagulation on a treated area of the skin according to a first effect, wherein according to the first effect, an area around a tip (302) of each needle (300) is coagulated separately rather than a space between the needles (300).

5. The skin treatment apparatus according to any of the preceding claims, wherein the plurality of needles (300) comprise at least 4 needles, preferably at least 9 needles.

6. The skin treatment apparatus according to any of the preceding claims, wherein the plurality of needles (300) is arranged in a net, in particular of quadratic shape.

7. The skin treatment apparatus according to any of the preceding claims, in particular according to claim 6, wherein the plurality of needles (300) is arranged such that each needle (300) has opposite polarity with its closest neighbouring needles.

8. The skin treatment apparatus according to any of the preceding claims, wherein each needle (300) is uninsulated at least in a dermis section (202).

9. The skin treatment apparatus according to any of the preceding claims, wherein a distance between a needle (300) and its closest neighbouring needle (300) is 1.3 - 3.0 mm.

10. The skin treatment apparatus according to any of the preceding claims, wherein the electricity has a high frequency of 0.5 - 10 MHz, preferably of 1 - 4 MHz, more preferably of 1.5 - 2.5 MHz.

11. The skin treatment apparatus according to any of the preceding claims, wherein the electricity has a voltage of 10 - 60V, preferably of 20 - 40 V.

12. The skin treatment apparatus according to any of the preceding claims, wherein a diameter of each needle (300) is between 0.25 - 0.3mm and a length of the needles (300) to be inserted into the skin is between 1 - 5mm, preferably between 0.1 - 1 mm.

13. The skin treatment apparatus according to any of the preceding claims, wherein the electricity is provided to the needles for 0.1 - 0.4 seconds, preferably for 0.1 - 0.2 seconds.

[Figure 1]

| Power Supply | RF Fenerator | Tip with Needles |
| Input | CPU | Output Display |

[Figure 2]

[Figure 3]

[Figure 4]

[Figure 5]

[Figure 6]

[Figure 7]

[Figure 8]

[Figure 9]

[Figure 10]

[Figure 11]